Europäisches Patentamt

European Patent Office

Office européen des brevets

(11) Publication number: **0 305 089**
**A1**

(12) **EUROPEAN PATENT APPLICATION**

(21) Application number: 88307461.9

(22) Date of filing: 11.08.88

(51) Int. Cl.4: **C07C 51/14 , C07C 57/30 ,**
**C07C 67/38 , C07C 69/24 ,**
**C07D 315/00**

(30) Priority: 22.08.87 GB 8719886

(43) Date of publication of application:
01.03.89 Bulletin 89/09

(84) Designated Contracting States:
BE CH DE FR GB IT LI NL

(71) Applicant: The British Petroleum Company
p.l.c.
Britannic House Moor Lane
London EC2Y 9BU(GB)

(72) Inventor: Alper, Howard
17 Winlock Crescent
Netean Ottawa K2G 3X5(CA)

(74) Representative: Denbigh, Keith Warwick et al
BP INTERNATIONAL LIMITED Patents
Division Chertsey Road
Sunbury-on-Thames Middlesex, TW16
7LN(GB)

(54) Process for the asymmetric production of carboxylic acid esters and/or carboxylic acids.

(57) A process for the asymmetric production of carboxylic acids or esters thereof is provided. The process comprises reacting a prochiral olefinic function and an alcoholic function or water with carbon monoxide, a single enantiomer of an optionally active compound, such as menthol, tartaric acid, tartaric acid esters, sugars, proteins and polypeptides, enzymes and chiral phosphines, and a catalyst. The catalyst comprises (a) a metal selected from palladium, rhodium ruthenium iridium and cobalt (preferably palladium) and copper. The reaction is optionally carried out in the presence of oxygen or air. The process is useful for making precursors for the pharmacologically important molecules ibuprofen and naproxen.

EP 0 305 089 A1

# PROCESS FOR THE ASYMMETRIC PRODUCTION OF CARBOXYLIC ACID ESTERS AND/OR CARBOXYLIC ACIDS

The present invention relates to a process for the asymmetric production of carboxylic acid esters and/or carboxylic acids by the catalysed reaction of carbon monoxide, optionally in the presence of oxygen, with a prochiral olefinic substrate. Typically the olefinic substrate can be an aryl substituted olefin e.g. p-isopropylstyrene where a branched product would give a chiral centre adjacent to the benzene ring, or an allylic or homoallylic alcohol where cyclisation results in a chiral centre adjacent to a lactone carbonyl function.

Processes for the production of carboxylic acid esters by reacting an olefin with carbon monoxide and an alcohol in the presence of a catalyst and in the presence or absence of oxygen are known. Representative of the published art are US Patent No 4303589, Belgian Patent No 877770, Japanese Patent Publication No 53040709 and US Patent No 3780074.

US Patent No 4303589 (Monsanto) describes a process for the production of carboxylates esters by (a) reacting internal olefins with carbon monoxide and an alcohol at 170 to 200° C and 1200-1800 psig in the presence of a cobalt catalyst and a pyridine promoter, (b) diluting the reaction mixture with a large amount of hydrocarbon to cause phase separation, (c) separating the ester from the other phase, which contains more than 90% of the cobalt catalyst and (d) recycling the catalyst to step (a).

Belgian Patent No 877770 describes the production of polycarboxylic esters by reacting an olefin containing at least two conjugated double bonds with carbon monoxide and an alcohol in the presence of a base and a palladium/copper catalyst.

Japanese Patent Publication No 53040709 describes the production of dicarboxylic acid diesters by reacting an olefin, carbon monoxide, oxygen and an alcohol in the presence of a catalyst containing (a) a palladium group metal or a compound thereof, (b) a copper salt and (c) a tertiary amine.

Finally, USP 3,780,075 describes the production of alkadienoic acid esters by reacting a 4-12 carbon acyclic conjugated aliphatic diolefin with a 1 to 20 carbon monohydroxy alcohol and carbon monoxide in the presence of zerovalent palladium and a phosphine activator at 80 to 160° C in the absence of oxygen.

Our copending European application publication No 105704 discloses a process for the production of a carboxylic acid ester which comprises reacting an unsaturated hydrocarbon with carbon monoxide and an alcohol in the presence of a protonic acid and as a catalyst (a) at least one of the metals palladium, rhodium, ruthenium, iridium and cobalt, and (b) copper.

In a more specific example of the above reaction, lactones can be produced by replacing the unsaturated hydrocarbon and the alcohol in the process of European application publication No 105704 by an unsaturated alcohol. This process being disclosed in our copending European application publication No 176370.

We have now found that by using a prochiral substrate, asymmetric induction at the resultant chiral centre can be effected by carrying out the hydrocarboxylation reaction in the presence of an optically active compound such as tartaric acid esters.

According to the present invention there is provided a process for preparing a chiral carboxylic acid or ester thereof either as a single enantiomer or as a mixture of enantiomers in which the presence of one enantiomer relative to the others which process comprises reacting a prochiral olefinic function and either water or an alcoholic function with carbon monoxide in the presence of a protonic acid other than nitric acid, a single enantiomer of an optically active compound chosen from (i) methanol, tartaric acid or a tartaric acid ester, (ii) sugars, (iii) proteins and polypeptides, (iv) enzymes and (v) chiral phosphines and a catalyst comprising (a) at least one of the metals palladium rhodium, ruthenium, iridium and cobalt and (b) copper,

The olefinic function may suitable be any such function that is prochiral and therefore by virtue of its reaction with carbon monoxide produces a chiral centre. Thus the olefin may suitably be an acyclic olefin containing from 4 to 30 carbon atoms per molecule or a cyclic olefin containing from 5 to 30 carbon atoms per molecule. The olefin may have either 1,2 or 3-olefinic carbon-carbon double bonds per molecular, which double bonds may be internal or terminal and may be conjugated or non-conjugated in olefins containing a plurality of carbon-carbon double bonds. Preferred olefins may be represented by the general formula RCH = CHR¹ wherein R and R¹ are independently either hydrogen, alkyl, alkenyl, alkadienyl, cycloalkyl, aryl, alkaryl, cycloalkenyl or cycloalkadienyl groups having 4 to 20 carbons atoms. R and R¹ may also be taken together form a cyclic system of 4 to 20 carbon atoms. Examples of such species include 1-butene, 2-butene, 1-pentene, 2-pentene, 1-hexene, 2-hexene, 3-hexene, 1-octene, 1-decene, cyclododecene, 2-methyl-l-undecene, styrene, 4-methylstyrene, 4-isopropylstyrene, and the like. Examples of diolefins falling within the preferred class include 1,4-pentadiene, 1,5-hexadiene, 4-vinyl cyclohexene, 1,7-octadiene, 1,9- decadiene and the like, whilst examples of triolefins include 1,5,9-cyclododecatriene, cycloheptatriene and the like.

A preferred embodiment of the present invention uses derivatives of styrene, examples of which are p-isopropylstyrene and p-isobutylstyrene or 2-vinyl-6-methoxy napthalene; the latter two compounds are precursors of ibuprofen and naproxen both of which are pharmacologically important molecules.

Considering next the optically active compound employed in the form of a single enantiomer, this is chosen from (i) menthol, tartaric acid or a tartaric acid ester, (ii) sugars, (iii) proteins and polypetides, (iv) enzymes and (v) chiral phosphines. If the first of these classes is employed it is preferred to use either tartaric acid or a lower (i.e. $C_1$ to $C_6$) alkyl ester thereof. Particularly suitable compound are either the D(+) or L(-) enantiomers of diethyl tartrate.

Examples of the other preferred classes which can be employed are cyclodextrins (sugars), bovine serum albumin (proteins), poly-L-leucine (polypeptides), lipase or horse liver alcohol dehydrogenase (enzymes) and (S) or (R) enantiomers of BINAP (chiral phosphine).

As regards the alcoholic function, monohydric or polyhydric alcohols may be employed. Suitable alcohols may be represented by the formula $R_2CHOH$ wherein R is independently hydrogen, alkyl, aryl or hydroxyalkyl, or the two groups R together form a ring. Suitably the alcohol is an alkanol. Examples of suitable alcohols include methanol, ethanol, propanols, butanols, pentanols, for example 2-ethylhexanol, benzyl alcohol and 1,4-butanediol. The amount of alcohol employed may suitably be at least the stoichiometric amount required to react with the unsaturated hydrocarbon. It is preferred, however, to employ a substantial excess of alcohol over the stoichiometric amount, the alcohol then performing the dual role of reactant and diluent for the reaction.

The alcoholic and olefinic function can be present in the same molecule i.e. it is possible to use an unsaturated alcohol as a reactant. In such cases it is preferred to use an unsaturated alcohol which yields a product which, under the conditions of the process, is capable of forming a lactone ring. Generally, unsaturated alcohols capable of forming 5-, 6- and 7-membered rings will be found most useful in the operation of such a process. Preferably the unsaturated alcohol is an allylic alcohols which may be a primary, secondary or tertiary allylic alcohol. Specific examples of unsaturated alcohols which may be employed in the process of the present invention are 3-buten-l-ol, trans-2-buten-l-ol, 3-buten-2-ol, 2-methyl-2-propen-1-ol, 2-methyl-3-buten-1-ol, 3-pentan-2-ol, 4-penten-2-ol, 4-penten-1-ol, 1-hexen-3-ol, 5-hexen-2-ol, cis-3-hexen-1-ol, cis-2-hexen-l-ol and trans-2-hexen-1-ol.

The carbon monoxide may be derived from any suitable source. Elevated pressures, suitably in the range from 2 to 250 psig above the autogenous pressure at the reaction temperature are preferably employed.

The protonic acid may be either a mineral acid, preferably hydrochloric acid or sulphuric acid (but not nitric acid) or an organic acid which may suitably be a carboxylic acid.

With regard to the catalyst, one or more of the metals palladium, rhodium, ruthenium, iridium and cobalt is employed as component (a). The metal(s) may either be in the form of the elemental metal(s), such as a finely divided powder, or in the form of a compound of the metal(s). Suitable compounds of the metal(s) include the chlorides, iodides, acetates, trifluoroacetates and nitrates, preferably the chlorides. It is preferred to use palladium either as a metal or as compound, e.g. palladium (II) chloride.

Copper, which constitutes component (b) of the catalyst may suitably be added as a cuprous or a cupric compound or as a mixture thereof. A wide variety of copper compounds may be used in the process of the invention. Examples of suitable copper compounds include copper (I) acetate, copper (II) acetylacetonate, copper (I) bromide, copper (I) chloride, copper (II) chloride, copper (I) iodide, copper (II) nitrate, copper (II) trifluoroacetate and the like.

As regards the ratios of the catalyst components, the molar ratio of copper component (b) to metal(s) component (a) may suitably be in the range 1:1 to 200:1, preferably from 2:1 to 50:1.

The molar ratio of unsaturated hydrocarbon to the metal(s) component (a) may suitably be in the range from 5:1 to 1000:1, preferably from 10:1 to 250:1.

Oxygen may be present or absent. However, it is preferred to operate in the presence of oxygen because by doing so the product yields can be improved. Oxygen may be supplied to the reaction either as essentially pure oxygen or admixed with other gases which are substantially inert under the reaction conditions. Air may conveniently be used as the source of oxygen. The oxygen pressure may suitably be the autogenous pressure at the reaction temperature employed. Alternatively elevated pressures may be employed if desired.

A solvent may be additionally employed if desired. The particular solvent employed may form a single phase with the alcohol reactant. Alternatively a solvent which is capable of forming a second liquid phase may be employed. The particular solvent employed should be inert the reaction conditions. Suitable solvents which form a single phase with the alcohol reactant include oxygenated hydrocarbons, for example tetrahydrofuran. Suitable solvents capable of forming a second liquid phase include aliphatic hydrocarbons, cycloaliphatic hydrocarbons, aromatic hydrocarbons, alkyl-substituted aromatic hydrocarbons or halogenated aliphatic or aromatic hydrocarbons. Examples of suitable solvents capable of forming a second liquid phase include benzene, toluene, hexane, cyclohexane, chlorobenzene, bromobenzene, a xylene dichloromethane, chloroform and 1,2-dichloroethane. It will be appreciated by those skilled in the art that the solvent should be chosen having regard to the difference in boiling points between the products of the reaction and the solvent so as to facilitate separation of the reaction mixture into its individual components. The amount of solvent based on the olefin reactant may vary over a wide range, suitably from 20 to 0.2, preferably from 5 to 1, volumes of solvent per volume of olefin reactant.

If, in a modification of the invention, the alcoholic function is replaced by water, provided that the amount of water is less than 8 mole equivalents based on the olefinic function and a solvent other than an alcohol is employed, then instead of carboxylic acid ester, there is formed the corresponding carboxylic acid.

Preferably the amount of water employed is less than 5, even more preferably about 1 mole equivalent based on the unsaturated hydrocarbon reactant.

When water is employed, any suitable solvent other than an alcohol may be used. Suitably solvents include ethers and hydrocarbons, for example paraffinic and aromatic hydrocarbons. Preferably the solvent is an ether. Examples of suitable ethers include tetrahydrofuran, dioxan, glymes and the crown ethers, of which tetrahydrofuran is preferred.

The process may suitably be operated at ambient temperature though elevated temperatures, for example in the range 20 to 150°C or even higher may be employed. The reaction time may vary over a wide range, suitably from about 30 minutes to 8 hours, though longer reaction times may be employed if desired.

The process may be carried out batchwise or continuously, preferably continuously.

The invention will now be described in greater detail by reference to the following Examples.

### Example 1 - Asymmetric Hydrocarboxylation of p-isopropylstyrene

Carbon monoxide was bubbled through a solution of water (1 ml) and tetrahydrofuran (30 ml). Palladium chloride (0.14 g, 0.78 mmol) was added, followed several minutes later by 1 ml of concentrated hydrochloric acid. After 10 minutes, cupric chloride (0.21g, 1.56 mmol) was added to the magnetically stirred solution and then oxygen bubbling was commenced. Finally, the alkene (7.8 mmol) and L(+) diethyl tartrate (0.65g, 3.0 mmol) were added, and the mixture was stirred overnight at room temperature. Distilled water (50 ml) was added and the product $(p(CH_3)_2CHC_6H_4CHCH_3COOH)$ was extracted with hexane (3 x 80 ml or ether), dried ($MgSO_4$), and concentrated. Pure acid was obtained in 76% yield and 18% R(-) enantiomeric excess by treatment with 1 M NaOH (50 ml), extraction with ether, and acidification to pH with 6 M HCl.

### Example 2 - Asymmetric Hydrocarboxylation of p-isobutylstyrene

The procedure used for p-isopropylstyrene was applied here, except that D(-)diethyl-tartrate was used as the added chiral ligand. Work up gave ibuprofen in 86% yield and 30% S(+) enantiomeric excess.

### Example 3 - Asymmetric Intramolecular Cyclisation of trans-2-hexen-1-ol

Carbon monoxide was bubbled through a stirred solution of tetrahydrofuran (30 ml). Palladium chloride (0.14g, 0.78 mmol) was added, followed by 0.5 ml of concentrated hydrochloric acid and cupric chloride (0.21g, 1.56 mmol). Oxygen was bubbled through the solution, trans-2-hexen-1-ol (7.8 mmol) and D(-)diethyl tartrate (0.65g, 3.0 mmol) were added and the mixture was stirred overnight at room temperature. The solution was filtered, and the filtrate was concentrated by rotary evaporation. Pure alpha-n-propyl-gamma-butyrolactone was obtained by thin-layer chromatography using 3:1 hexane-ether. Yield:50%. % enantiomeric excess S(+) = 24%.

Example 4 - Asymmetric Intramolecular Cyclisation of 3-buten-1-ol

The procedure of Example 3 was used, with D(+) diethyl tartrate as the ligand. Yield of alpha-methyl-gamma-butyrolactone = 44%. % enantiomeric excess R(-) = 46%.

Examples 5-10

Example 3 was repeated using 7.8 mmol of $CH_3CH = CHCH_2OH$ and the appropriate amount of the particular optically active compound. The % enantiomeric excess of the major enantiomer is shown in the Table.

Examples 11-15

Example 1 was repeated using 7.8 mmol of the appropriate styrene or napthalene derivative. The particular optically active compound used and the amount is given in the Table. Again the % enantiomeric excess of the major enantiomer was recorded.

TABLE

| Example | Olefin (1) | Optically Active Compound (2) | Ratio $\frac{(1)}{(2)}$ | % Excess of Enantiomer |
|---|---|---|---|---|
| 5 | $CH_3CH = CHCH_2OH$ | d-menthol | 7.8 | 21 R |
| 6 | | (-)dimethyl-2,3-0 benzylidene L-tartrate | 10 | 33 S |
| 7 | | (-)S-BINAP | 24 | 40 S |
| 8 | | (-)S-BINAP | 98 | 21 S |
| 9 | | (+)R-BINAP | 50 | 36 R |
| 10 | | (-)Poly-L-leucine | 50 mg* | 61 S |
| 11 | p-methylstyrene | d-menthol | 2.6 | 40 R |
| 12 | | L-diethyl tartrate | 7.6 | 36 S |
| 13 | p-isobutylstyrene | d-menthol | 2.6 | 55 R |
| 14 | | D-diethyl tartrate | 2.6 | 31 R |
| 15 | 2-vinyl-6-methoxy naphthalene | d-menthol | 40 | 56 R |

*50 mg of (-)poly-L-leucine used in this experiment.

## Claims

1. A process for preparing a chiral carboxylic acid or ester thereof, either as a single enantiomer or as a mixture of enantiomers in which the presence of one enantiomer is enhanced relative to the others which process comprises reacting a prochiral olefinic function and either water or an alcoholic function with carbon monoxide in the presence of a protonic acid other than nitric acid, a single enantiomer of an optically active compound chosen from (1) menthol, tartaric acid or a tartaric acid ester, (2) sugars, (3) proteins and polypeptides, (4) enzymes and (v) chiral phosphines, and a catalyst comprising (a) at least one of the metals palladium, rhodium, ruthenium, indium and cobalt and (b) copper.

2. A process as claimed in claim 1 wherein an alcoholic function is used and wherein the prochiral olefinic function and the alcoholic function are provided by an unsaturated alcohol.

3. A process as claimed in claim 2 wherein the unsaturated alcohol is one yielding a product capable of forming a lactone ring.

4. A process as claimed in claim 1 wherein the optically active compound is a $C_1$ to $C_6$ alkyl ester of tartaric acid.

**5.** A process as claimed in claim 4 wherein the $C_1$ to $C_5$ alkyl ester of tartaric acid is D(+) or L(-) diethyl tartrate.

**6.** A process as claimed in claim 1 where component (a) of the catalyst is either palladium metal or a palladium compound.

**7.** A process as claimed in claim 1 which is carried out in the presence of essentially pure oxygen or air.

**8.** A process as claimed in claim 1 wherein the olefinic function is a derivative of styrene or 2-vinyl, 6-methoxy napthalene.

**9.** A process as claimed in claim 1 wherein the styrene is either p-isopropylbenzene or p-isobutylbenzene.

**10.** A process as claimed in claim 1 wherein the optically active compound is selected from cyclodextrins, bovine serum albumin, poly-L-leucine, horse liver dehydrogenase or the (S) or (R) enantiomer of BINAP.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | EP-A-0 081 149 (CONSIGLIO NAZIONALE DELLE RICERCHE) * Claims 3,7; pages 15-17, 29-31 * | 1,6,8 | C 07 C 51/14 C 07 C 57/30 C 07 C 67/38 C 07 C 69/24 C 07 D 315/00 |
| Y | US-A-4 612 390. (SHUM et al.) * Claims 1,6-8 * | 1,6 | |
| Y | PATENT ABSTRACTS OF JAPAN, vol. 4, no. 30 (C-2)[512], 15th March 1980; & JP-A-55 7206 (KOGYO GIJUTSUIN) 19-01-1980 | 1,6,8 | |
| D,A | EP-A-0 105 704 (THE BRITISH PETROLEUM CO.) * Claims 1-6; page 3, lines 2-16; page 4, lines 18-20,28 - page 5, lines 1,8-10 * | 1,6-8 | |

TECHNICAL FIELDS
SEARCHED (Int. Cl.4)

C 07 C 51/00
C 07 C 53/00
C 07 C 57/00
C 07 C 67/00
C 07 C 69/00

The present search report has been drawn up for all claims

| Place of search | Date of completion of the search | Examiner |
|---|---|---|
| THE HAGUE | 17-11-1988 | KLAG M.J. |